(19) Europäisches Patentamt
European Patent Office
Office européen des brevets

(11) Veröffentlichungsnummer: **0 207 411**
**B1**

# (12) EUROPÄISCHE PATENTSCHRIFT

(45) Veröffentlichungstag der Patentschrift: **18.01.89**

(51) Int. Cl.⁴: **C07D 213/65,** A01N 43/40

(21) Anmeldenummer: **86108499.4**

(22) Anmeldetag: **21.06.86**

## (54) O-substituierte 3-Oxypyridiniumsalze, Verfahren zu ihrer Herstellung und ihre Verwendung als Fungizide für den Pflanzenschutz.

(30) Priorität: **27.06.85 DE 3522905**

(43) Veröffentlichungstag der Anmeldung: **07.01.87 Patentblatt 87/2**

(45) Bekanntmachung des Hinweises auf die Patenterteilung: **18.01.89 Patentblatt 89/3**

(84) Benannte Vertragsstaaten: **AT BE CH DE FR GB IT LI NL SE**

(56) Entgegenhaltungen:
**DE-C- 532 396**
**US-A- 2 728 775**

(73) Patentinhaber: **BASF Aktiengesellschaft, Carl-Bosch-Strasse 38, D-6700 Ludwigshafen(DE)**

(72) Erfinder: **Schulz, Günter, Dr., Carl-Bosch-Strasse 96, D-6700 Ludwigshafen(DE)**
Erfinder: **Ammermann, Eberhard, Dr., Sachsenstrasse 3, D-6700 Ludwigshafen(DE)**
Erfinder: **Pommer, Ernst-Heinrich, Dr., Berliner Platz 7, D-6703 Limburgerhof(DE)**

EP 0 207 411 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft neue O-substituierte 3-Oxypyridiniumsalze, Verfahren zu ihrer Herstellung und fungizide Mittel für den Pflanzenschutz, die diese Verbindungen enthalten.

Es ist bekannt, N-Trichlormethylthio-tetrahydrophthalimid als Fungizid zu verwenden (Chemical Week, June 21, 1972, Seite 46). Seine Wirkung ist jedoch schlecht.

Es ist ferner bekannt, das n-Cetyl-(3-hydroxypyridinium)-chlorid als Fungizid zu verwenden (US-A 2 728 775). Hierdurch werden die erfindungsgemäßen Salze nicht nahegelegt.

Es wurde gefunden, daß O-substituierte 3-Oxypyridiniumsalze der Formel

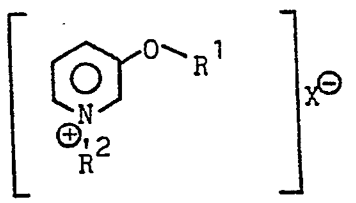 I,

in der

$R^1$ einen gegebenenfalls durch Alkoxy oder Halogen substituierten Alkyl-, Alkenyl- oder Alkinylrest mit insgesamt 8 – 20 C-Atomen oder eine der Gruppen

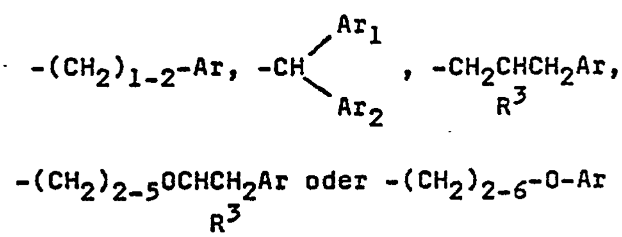

oder

bedeutet, wobei Ar, $Ar_1$ und $Ar_2$ für 1- und 2-Naphthyl, Biphenyl oder Phenyl steht und der Phenylrest durch F, Cl, Br, $NO_2$, $CF_3$, CN, $C_{1-13}$-Alkyl, $C_{2-4}$-Alkenyl oder $C_{1-2}$-Alkoxy substituiert sein kann,

$R^3$ H, $C_{1-2}$-Alkyl, $C_{3-4}$-Alkenyl oder $C_1$–$C_5$-Alkoxy bedeutet und

$R^2$ einen gegebenenfalls durch $C_1$–$C_4$-Alkoxy oder Halogen substituierten Alkyl-, Alkenyl- oder Alkinylrest mit 1 bzw. 2 oder 3 bis 8 Kohlenstoffatomen oder eine der Gruppen

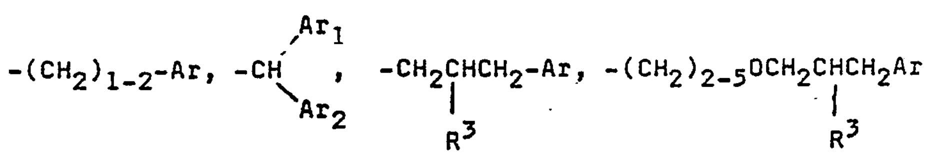

oder $-(CH_2)_{2-6}$-O-Ar bedeutet, wobei Ar, $Ar_1$ und $Ar_2$ für 1- und 2-Naphthyl, Biphenyl oder Phenyl steht und der Phenylrest durch F, Cl, Br, $NO_2$, $CF_3$, CN, $C_{1-13}$-Alkyl, $C_{2-4}$-Alkenyl oder $C_{1-2}$-Alkoxy substituiert sein kann, und

$X^-$ $F^-$, $Cl^-$, $Br^-$ oder $I^-$ oder ein Anion, das Äquivalent einer nicht phytotoxischen Säure ist, bedeutet, eine gute fungizide Wirksamkeit gegenüber phytopathogenen Pilzen zeigen.

$R^1$ bedeutet beispielsweise gerades oder verzweigtes, gegebenenfalls durch 2–5 Cl- oder F-Atome substituiertes $C_8$–$C_{18}$-Alkyl, insbesondere $C_{10}$–$C_{18}$-Alkyl, z.B. Octyl, Nonyl, Decyl, Undecyl, Dodecyl, Tridecyl,Tetradecyl, Pentadecyl, Hexadecyl, Heptadecyl, Octadecyl oder die Gruppe $-C_{2-10}$Alkyl-O-$C_{5-18}$Alkyl mit insgesamt 10 – 20 C-Atomen und gegebenenfalls 1 bis 2 Doppelbindungen oder einer Dreifachbindung, die durch mindestens eine $CH_2$-Gruppe von O getrennt ist, z.B. 5-Decenyl, 8-Octadecenyl, 5-Dodecinyl, oder die Reste

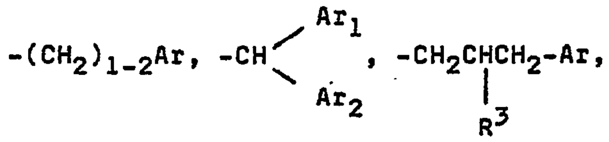

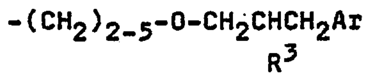 oder $-(CH_2)_{2-6}OAr$,

wobei Ar, $Ar_1$ und $Ar_2$ für 1- oder 2-Naphthyl, Biphenyl, Phenyl, einfach bis dreifach substituiertes Phenyl, 4-Fluorphenyl, 3- oder 4-Chlorphenyl, 4-Bromphenyl, 3,4-Dichlorphenyl, 2,4-Dichlorphenyl, 2,6-Dichlorphenyl, 2,4,6-Trichlorphenyl, 2-Methyl-4-Chlorphenyl, 4-Methoxyphenyl, 4-Ethoxyphenyl, 4-Methylphenyl, 4-Nitrophenyl, 4-tert.-Butylphenyl, 4-Dodecylphenyl, 4-Trifluormethylphenyl, 2-Trifluormethyl-4-Chlorphenyl, 2,4-Dimethylphenyl, 2,4,6-Trimethylphenyl und 4-Cyanophenyl steht,
$R^3$ bedeutet beispielsweise Wasserstoff, Methyl oder Ethyl,
$R^2$ bedeutet beispielsweise
Methyl, Ethyl, Propyl, Isopropyl, n-Butyl, Isobutyl, n-Pentyl, Isopentyl, n-Hexyl, Allyl, 2-Chlorpropenyl, 2-Brompropenyl, 3-Chlorpropenyl, 2-Buten-1-yl, 3-Methyl-2-buten-1-yl, 2-Methylpropenyl, 3-Chlor-2-buten-1-yl, Propargyl oder die Reste

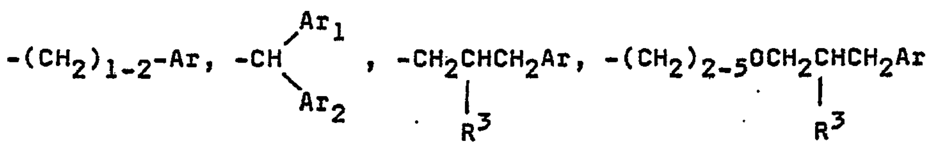

oder $-(CH_2)_{2-6}OAr$ bedeutet,

oder $-(CH_2)_{2-6}OAr$ bedeutet,
wobei Ar, $Ar_1$ und $Ar_2$ für 1- oder 2-Naphthyl, Biphenyl, Phenyl, einfach bis dreifach substituiertes Phenyl, 4-Fluorphenyl, 3- oder 4-Chlorphenyl, 4-Bromphenyl, 3,4-Dichlorphenyl, 2,4-Dichlorphenyl, 2,6-Dichlorphenyl, 2,4,6-Trichlorphenyl, 2-Methyl-4-Chlorphenyl, 4-Methoxyphenyl, 4-Ethoxyphenyl, 4-Methylphenyl, 4-Nitrophenyl, 4-tert.-Butylphenyl, 4-Dodecylphenyl, 4-Trifluormethylphenyl, 2-Trifluormethyl-4-Chlorphenyl, 2,4-Dimethylphenyl, 2,4,6-Trimethylphenyl oder 4-Cyanophenyl steht, und
$X^{\ominus}$ bedeutet $Cl^{\ominus}$, $Br^{\ominus}$, $I^{\ominus}$ oder ein Anion, das ein Äquivalent einer nicht phytotoxischen Säure ist wie Phenylsulfonsäure, p-Methylphenylsulfonsäure oder ein Äquivalent des Schwefelsäure-Anions.

Man erhält die O-substituierten 3-Oxypyridiniumsalze der Formel I durch Umsetzung von Verbindungen der Formel
$R^2X$ II,
in der $R^2$ und X die oben angegebenen Bedeutungen haben mit einem O-substituierten 3-Oxypyridin-Derivat der Formel

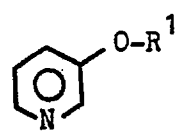 III,

in der $R^1$ die obengenannte Bedeutung hat.

Die Reaktion wird ohne Verdünnungsmittel in Substanz oder gegebenenfalls in Gegenwart eines inerten Lösungs- oder Verdünnungsmittels bei einer Temperatur zwischen 20 und 150°C vorzugsweise zwischen 50 und 150°C durchgeführt. Der Ausgangsstoff der Formel III wird zweckmäßig mit einer bis zu 10fachen Menge (molar) des Alkylierungsmittels der Formel II bezogen auf den Ausgangsstoff III umgesetzt.

Als bevorzugte, gegenüber den Reaktionsteilnehmern inerte Lösungsmittel oder Verdünnungsmittel können beispielsweise aliphatische oder aromatische gegebenenfalls halogenierte Kohlenwasserstoffe, wie Pentan, Cyclohexan, Heptan, Benzol, Toluol, Xylol, Chlorbenzol, Dichlorbenzole, aliphatische Ketone, wie Aceton, Methylethylketon, Diethylketon oder Cyclopentanon, Ether wie Diethylether, Methyl-tert.-butylether, Dimethoxyethan, Tetrahydrofuran oder Dioxan, Ester wie Essigsäureethylester, Nitrile wie Acetonitril, Amide wie Dimethylformamid, Dimethylacetamid und N-Methylpyrrolidon, oder Gemische dieser Lösungsmittel verwendet werden.

Die Ausgangsstoffe der Formel II und III sind weitgehend bekannt und z. T. kommerziell zugänglich, bzw. sie lassen sich analog zu literaturbekannten Beispielen herstellen (für III z. B. entsprechend Chem. Ber. 116, 2394 (1983)).

Die neuen O-substituierten 3-Oxypyridiniumsalze besitzen gegebenenfalls in $R^1$ und/oder $R^2$ ein chirales Kohlenstoffatom, wenn $R^3$ nicht gleich Wasserstoff ist. Nach den üblichen Methoden können die optisch reinen Enantiomeren bzw. die Diastereomeren erhalten werden. Die vorliegende Erfindung erfaßt sowohl diese Verbindungen in reiner Form als auch ihre Mischungen. Es sind sowohl die reinen Enantiomeren bzw. die einheitlichen Diastereomeren als auch die bei der Synthese üblicherweise anfallenden Mischungen wirksam.

Die folgenden Beispiele erläutern die Herstellung der neuen Verbindungen:

Herstellungsbeispiel

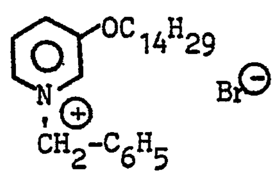

a) **Ausgangsmaterial**

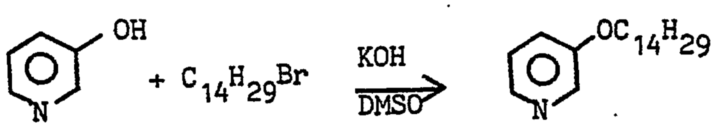


A

100 g (1,04 mol) 3-Hydroxypyridin werden in 500 ml Dimethylsulfoxid (DMSO) mit 87,4 g (1,56 mol) KOH-Pulver unter Stickstoff bei Raumtemperatur (20°C) gerührt. Nach ca. 30 Min. trägt man 360 g (1,3 mol) Tetradecylbromid tropfenweise ein und rührt 4 h bei Raumtemperatur nach. Das Reaktionsgemisch wird in 1 l Wasser gegossen und dreimal mit je 500 ml Methylenchlorid extrahiert. Die vereinigten Extrakte werden zweimal mit 1 l Wasser gewaschen, mit $Na_2SO_4$ getrocknet und eingedampft. Filtration über Kieselgel mit n-Pentan und Methylchlorid führt zu 144 g Produkt A , Fp. 36°C.

b) **Pyridiniumsalzbildung**

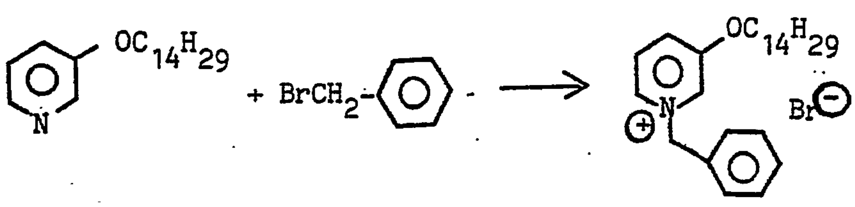

A B

11,5 g (0,04 mol) 3-Tetradecyloxypyridin A werden mit 5 ml (0,04 mol) Benzylbromid 20 Min. bei 100 – 120°C gerührt. Nach Abkühlen auf Raumtemperatur wird in Pentan suspendiert, abgesaugt, mit Pentan nachgewaschen und getrocknet: 16,8 g Pyridiniumsalz B, Fp. 129°C (Verbindung Nr. 23).

Herstellungsbeispiel 2

a) Ausgangsmaterial

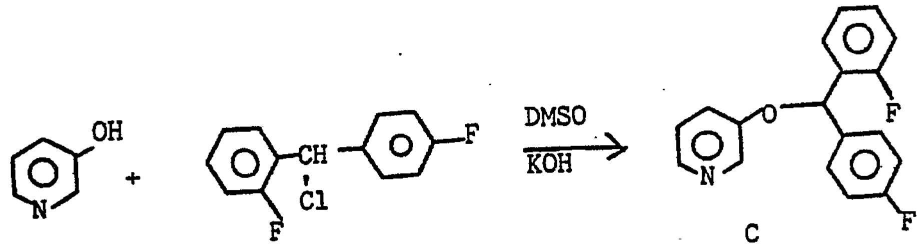


C

19 g 3-Hydroxpyridin werden in 200 ml DMSO mit 33,6 g KOH-Pulver verrührt. Nach 30 Min. werden bei Raumtemperatur 59,6 g 2,4'-Difluor-Benzhydrylchlorid tropfenweise eingetragen. Nach 40 Min. wird das Reaktionsgemisch in Eiswasser gegossen und mit Ether (dreimal 300 ml) extrahiert. Das Produkt wird durch vorsichtiges Zusetzen von conc. $H_2SO_4$ als Hydrosulfat gefällt. Man dekantiert den Ether und setzt das Produkt mit Hydrogencarbonat frei, nimmt in Methylenchlorid auf, trocknet mit $Na_2SO_4$ und dampft ein: 38,5 g C als Öl.

b) Bildung des Pyridiniumsalzes

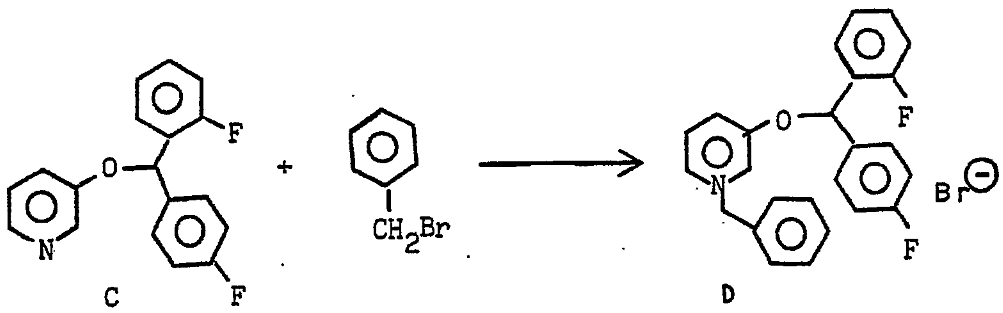

C D

10 g C werden in 100 ml Dimethylformamid (DMF) mit 5,8 g Benzylbromid verrührt. Nach ca. 1 h wird DMF im Vakuum abdestilliert, der Rückstand in Pentan suspendiert und abgesaugt: 14,4 g Verbindung D, Fp. 173°C (Verbindung Nr. 165).

Die in der folgenden Tabelle aufgelisteten Verbindungen sind auf entsprechende Weise herstellbar:

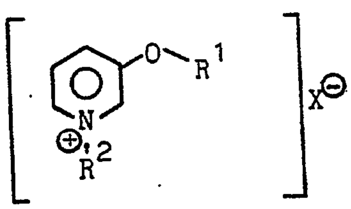

I,

| Nr. | $R^1$ | $R^2$ | X | Fp. [°C] |
|---|---|---|---|---|
| 1 | $n-C_{10}H_{21}$ | $CH_3$ | Cl | |
| 2 | $n-C_{10}H_{21}$ | $CH_3$ | Br | |
| 3 | $n-C_{10}H_{21}$ | $CH_3$ | I | |
| 4 | $n-C_{12}H_{25}$ | $CH_3$ | Br | |
| 5 | $n-C_{12}H_{25}$ | $CH_3$ | I | 64 – 71 |
| 6 | $n-C_{12}H_{25}$ | $-CH_2-CH=CH_2$ | Br | 69 |
| 7 | $n-C_{12}H_{25}$ | $-CH_2-C_6H_5$ | Br | 65 – 65 |
| 8 | $n-C_{12}H_{25}$ | $-CH_2-C_6H_5$ | Cl | |
| 9 | $n-C_{12}H_{25}$ | $-CH_2-(2-Cl-C_6H_4)$ | Cl | 89 |
| 10 | $n-C_{12}H_{25}$ | $-CH_2-(3-Cl-C_6H_4)$ | Cl | |
| 11 | $n-C_{12}H_{25}$ | $-CH_2-(4-Cl-C_6H_4)$ | Cl | 93 |
| 12 | $n-C_{12}H_{25}$ | $-CH_2CH_2O-(2-Cl-C_6H_4)$ | Br | 94 |
| 13 | $n-C_{12}H_{25}$ | $-CH_2CH_2O-(3-Cl-C_6H_4)$ | Br | 146 |
| 14 | $n-C_{12}H_{25}$ | $-CH_2CH_2O-(4-Cl-C_6H_4)$ | Br | 135 |
| 15 | $n-C_{12}H_{25}$ | $-CH_2-CH=CHCl$ | Cl | |
| 16 | $n-C_{12}H_{25}$ | $-CH_2-C\equiv CH$ | Br | |
| 17 | $n-C_{12}H_{25}$ | $-CH_2CH=CH-CH_3$ | Cl | |
| 18 | $n-C_{12}H_{25}$ | $-CH_2CCl=CH_2$ | Br | |
| 19 | $n-C_{12}H_{25}$ | $-CH_2CCl=CCl_2$ | Br | |
| 20 | $n-C_{14}H_{29}$ | $CH_3$ | Br | |
| 21 | $n-C_{14}H_{29}$ | $CH_3$ | Cl | |
| 22 | $n-C_{14}H_{29}$ | $CH_3$ | I | 91 – 95 |
| 23 | $n-C_{14}H_{29}$ | $-CH_2-C_6H_5$ | Br | 129 |
| 24 | $n-C_{14}H_{29}$ | $-CH_2-C_6H_5$ | Cl | |
| 25 | $n-C_{14}H_{29}$ | $-CH_2-(2-Cl-C_6H_4)$ | Cl | 92 |
| 26 | $n-C_{14}H_{29}$ | $-CH_2-(3-Cl-C_6H_4)$ | Cl | |
| 27 | $n-C_{14}H_{29}$ | $-CH_2-(4-Cl-C_6H_4)$ | Cl | 115 |
| 28 | $n-C_{14}H_{29}$ | $-CH_2CH_2O-(2-Cl-C_6H_4)$ | Br | 97 |
| 29 | $n-C_{14}H_{29}$ | $-CH_2CH_2O-(3-Cl-C_6H_4)$ | Br | 144 |
| 30 | $n-C_{14}H_{29}$ | $-CH_2CH_2O-(4-Cl-C_6H_4)$ | Br | 133 |
| 31 | $n-C_{14}H_{29}$ | $-CH_2-CH=CH_2$ | Cl | |
| 32 | $n-C_{14}H_{29}$ | $-CH_2-CH=CH_2$ | Br | 83 |
| 33 | $n-C_{14}H_{29}$ | $-CH_2-CH=CHCl$ | Cl | |
| 34 | $n-C_{14}H_{29}$ | $-CH_2-CH=CH-CH_3$ | Cl | |
| 35 | $n-C_{14}H_{29}$ | $-CH_2-CCl=CCl_2$ | Cl | |
| 36 | $n-C_{14}H_{29}$ | Propargyl | Br | |
| 37 | $n-C_{14}H_{29}$ | Propargyl | Cl | |
| 38 | $n-C_{16}H_{33}$ | $CH_3$ | Br | |
| 39 | $n-C_{16}H_{33}$ | $CH_3$ | Cl | |
| 40 | $n-C_{16}H_{33}$ | $CH_3$ | I | 96 – 101 |
| 41 | $n-C_{16}H_{33}$ | $-CH_2C_6H_5$ | Br | 57 – 60 |
| 42 | $n-C_{16}H_{33}$ | $-CH_2C_6H_5$ | Cl | |
| 43 | $n-C_{16}H_{33}$ | $-CH_2-(2-Cl-C_6H_4)$ | Cl | |
| 44 | $n-C_{16}H_{33}$ | $-CH_2-(3-Cl-C_6H_4)$ | Cl | |
| 45 | $n-C_{16}H_{33}$ | $-CH_2-(4-Cl-C_6H_4)$ | Cl | |
| 46 | $n-C_{16}H_{33}$ | $-CH_2CH_2O-(2-Cl-C_6H_4)$ | Br | |
| 47 | $n-C_{16}H_{33}$ | $-CH_2CH_2O-(3-Cl-C_6H_4$ | Br | |

| Nr. | $R^1$ | $R^2$ | X | Fp. [°C] |
|---|---|---|---|---|
| 48 | $n-C_{16}H_{33}$ | $-CH_2CH_2O-(4-Cl-C_6H_4)$ | Br | |
| 49 | $n-C_{16}H_{33}$ | $-CH_2CH_2O-C_6H_5$ | Br | |
| 50 | $n-C_{14}H_{29}$ | $-CH_2CH_2O-C_6H_5$ | Br | |
| 51 | $n-C_{12}H_{25}$ | $-CH_2CH_2O-C_6H_5$ | Br | |
| 52 | $n-C_{10}H_{21}$ | $-CH_2CH_2O-C_6H_5$ | Cl | |
| 53 | $n-C_{16}H_{33}$ | $-CH_2-CH=CH_2$ | Cl | |
| 54 | $n-C_{16}H_{33}$ | $-CH_2-CH=CHCl$ | Cl | |
| 55 | $n-C_{16}H_{33}$ | $-CH_2CH=CH-CH_3$ | Cl | |
| 56 | $n-C_{16}H_{33}$ | $-CH_2-CCl=CCl_2$ | Cl | |
| 57 | $n-C_{16}H_{33}$ | Propargyl | Br | |
| 58 | $n-C_{18}H_{37}$ | $CH_3$ | Br | |
| 59 | $n-C_{18}H_{37}$ | $CH_3$ | Cl | |
| 60 | $n-C_{18}H_{37}$ | $CH_3$ | I | 89 |
| 61 | $n-C_{18}H_{37}$ | $-CH_2-C_6H_5$ | Br | 71–74 |
| 62 | $n-C_{18}H_{37}$ | $-CH_2-C_6H_5$ | Cl | |
| 63 | $n-C_{18}H_{37}$ | $-CH_2-(2-Cl-C_6H_4)$ | Cl | |
| 64 | $n-C_{18}H_{37}$ | $-CH_2-(3-Cl-C_6H_4)$ | Cl | |
| 65 | $n-C_{18}H_{37}$ | $-CH_2-(4-Cl-C_6H_4)$ | Cl | |
| 66 | $n-C_{18}H_{37}$ | $-CH_2CH_2O-C_6H_5$ | Br | |
| 67 | $n-C_{18}H_{37}$ | $-CH_2CH_2O-(2-Cl-C_6H_4)$ | Br | |
| 68 | $n-C_{18}H_{37}$ | $-CH_2CH_2O-(3-Cl-C_6H_4)$ | Br | |
| 69 | $n-C_{18}H_{37}$ | $-CH_2CH_2O-(4-Cl-C_6H_4)$ | Br | |
| 70 | $n-C_{18}H_{37}$ | $-CH_2-CH=CH_2$ | Br | |
| 71 | $n-C_{18}H_{37}$ | $-CH_2-CH=CHCl$ | Cl | |
| 72 | $n-C_{18}H_{37}$ | $-CH_2CH=CH-CH_3$ | Cl | |
| 73 | $n-C_{18}H_{37}$ | $-CH_2-CCl=CCl_2$ | Cl | |
| 74 | $n-C_{18}H_{37}$ | Propargyl | Br | |
| 75 | $n-C_{20}H_{41}$ | $-CH_3$ | Br | |
| 76 | $n-C_{20}H_{41}$ | $-CH_3$ | I | |
| 77 | $n-C_{20}H_{41}$ | $-CH_3$ | Cl | |
| 78 | $n-C_{20}H_{41}$ | $-CH_2C_6H_5$ | Cl | |
| 79 | $n-C_{20}H_{41}$ | $-CH_2C_6H_5$ | Br | |
| 80 | $-C_{20}H_{41}$ | $-CH_2CH_2O-C_6H_5$ | Br | |
| 81 | $-(CH_2)_4-O-(CH_2)_7-CH_3$ | $-CH_3$ | Br | |
| 82 | $-(CH_2)_4-O-(CH_2)_7-CH_3$ | $-CH_3$ | I | |
| 83 | $-(CH_2)_4-O-(CH_2)_7-CH_3$ | $-CH_2-C_6H_5$ | Cl | |
| 84 | $-(CH_2)_4-O-(CH_2)_7-CH_3$ | $-CH_2-C_6H_5$ | Br | 79 |
| 85 | $-(CH_2)_4-O-(CH_2)_7-CH_3$ | $-CH_2CH_2O-C_6H_5$ | Br | |
| 86 | $-(CH_2)_4-O-(CH_2)_7-CH_3$ | $-CH_2-CH=CH_2$ | Br | |
| 87 | $-(CH_2)_4-O(CH_2)_{10}CH_3$ | $-CH_3$ | Br | |
| 88 | $-(CH_2)_4-O(CH_2)_{10}CH_3$ | $-CH_3$ | I | 105 |
| 89 | $-(CH_2)_4-O(CH_2)_{10}CH_3$ | $-CH_2-C_6H_5$ | Cl | |
| 90 | $-(CH_2)_4-O(CH_2)_{10}CH_3$ | $-CH_2-C_6H_5$ | Br | |
| 91 | $-(CH_2)_4-O(CH_2)_{10}CH_3$ | $-CH_2CH_2O-C_6H_5$ | Br | |
| 92 | $-(CH_2)_4-O(CH_2)_{10}CH_3$ | $-CH_2-CH=CH_2$ | Cl | |
| 93 | $-(CH_2)_5-O-CH_2CH(C_2H_5)CH_2CH(CH_3)_2$ | $-CH_3$ | Br | |
| 94 | $-(CH_2)_5-O-CH_2CH(C_2H_5)CH_2CH(CH_3)_2$ | $-CH_3$ | I | |

| Nr. | $R^1$ | $R^2$ | X | Fp. [°C] |
|---|---|---|---|---|
| 95 | $-(CH_2)_5-O-CH_2CH(C_2H_5)CH_2CH(CH_3)_2$ | $-CH_2-CH=CH_2$ | Br | |
| 96 | $-(CH_2)_5-O-CH_2CH(C_2H_5)CH_2CH(CH_3)_2$ | $-CH_2C_6H_5$ | Br | Öl |
| 97 | $-(CH_2)_5-O-CH_2CH(C_2H_5)CH_2CH(CH_3)_2$ | $-CH_2CH_2O-C_6H_5$ | Br | |
| 98 | $-(CH_2)_6-O-CH_2CH(CH_3)CH_2CH(CH_3)_2$ | $CH_3$ | I | |
| 99 | $-(CH_2)_6-O-CH_2CH(CH_3)CH_2CH(CH_3)_2$ | $CH_3$ | Br | |
| 100 | $-(CH_2)_6-O-CH_2CH(CH_3)CH_2CH(CH_3)_2$ | $-CH_2-C_6H_5$ | Br | |
| 101 | $-(CH_2)_6-O-CH_2CH(CH_3)CH_2CH(CH_3)_2$ | $-CH_2-C_6H_5$ | Cl | |
| 102 | $-(CH_2)_6-O-CH_2CH(CH_3)CH_2CH(CH_3)_2$ | $-CH_2CH_2O-C_6H_5$ | Br | |
| 103 | $-(CH_2)_6-O-CH_2CH(CH_3)CH_2CH(CH_3)_2$ | $-CH_2CH=CH_2$ | Br | |
| 104 | $-(CH_2)_6-O-CH(CH_3)CH_2CH_2CH(C_2H_5)_2$ | $-CH_3$ | Br | |
| 105 | $-(CH_2)_6-O-CH(CH_3)CH_2CH_2CH(C_2H_5)_2$ | $-CH_3$ | I | |
| 106 | $-(CH_2)_6-O-CH(CH_3)CH_2CH_2CH(C_2H_5)_2$ | $-CH_2-C_6H_5$ | Br | |
| 107 | $-(CH_2)_6-O-CH(CH_3)CH_2CH_2CH(C_2H_5)_2$ | $-CH_2-C_6H_5$ | Cl | |
| 108 | $-(CH_2)_6-O-CH(CH_3)CH_2CH_2CH(C_2H_5)_2$ | $-CH_2CH_2OC_6H_5$ | Br | |
| 109 | $-(CH_2)_6-O-CH(CH_3)CH_2CH_2CH(C_2H_5)_2$ | $-CH_2CH=CH_2$ | Br | |
| 110 | $-(CH_2)_5-O-CH_2CH_2CH(C_2H_5)(CH_2)_3CH_3$ | $-CH_3$ | I | |
| 111 | $-(CH_2)_5-O-CH_2CH_2CH(C_2H_5)(CH_2)_3CH_3$ | $-CH_3$ | Br | |
| 112 | $-(CH_2)_5-O-CH_2CH_2CH(C_2H_5)(CH_2)_3CH_3$ | $-CH_2C_6H_5$ | Cl | |
| 113 | $-(CH_2)_5-O-CH_2CH_2CH(C_2H_5)(CH_2)_3CH_3$ | $-CH_2C_6H_5$ | Br | |
| 114 | $-(CH_2)_5-O-CH_2CH_2CH(C_2H_5)(CH_2)_3CH_3$ | $-CH_2CH=CH_2$ | Br | |
| 115 | $-(CH_2)_5-O-CH_2CH_2CH(C_2H_5)(CH_2)_3CH_3$ | $-CH_2CH_2O-C_6H_5$ | Br | |
| 116 | $-(CH_2)_6-O-(CH_2)_2CH(CH_3)(CH_2)_3CH(CH_3)_2$ | $CH_3$ | Br | |
| 117 | $-(CH_2)_6-O-(CH_2)_2CH(CH_3)(CH_2)_3CH(CH_3)_2$ | $CH_3$ | I | |
| 118 | $-(CH_2)_6-O-(CH_2)_2CH(CH_3)(CH_2)_3CH(CH_3)_2$ | $-CH_2C_6H_5$ | Cl | |
| 119 | $-(CH_2)_6-O-(CH_2)_2CH(CH_3)(CH_2)_3CH(CH_3)_2$ | $-CH_2C_6H_5$ | Br | Öl |
| 120 | $-(CH_2)_6-O-(CH_2)_2CH(CH_3)(CH_2)_3CH(CH_3)_2$ | $-CH_2CH=CH_2$ | Br | |
| 121 | $-(CH_2)_6-O-(CH_2)_2CH(CH_3)(CH_2)_3CH(CH_3)_2$ | $-CH_2CH_2O-C_6H_5$ | Br | |
| 122 | $-(CH_2)_4-O-(CH_2)_2CH(CH_3)(CH_2)_3CH(CH_3)(CH_2)_3CH(CH_3)_2$ | $CH_3$ | Br | |
| 123 | $-(CH_2)_4-O-(CH_2)_2CH(CH_3)(CH_2)_3CH(CH_3)(CH_2)_3CH(CH_3)_2$ | $CH_3$ | I | |
| 124 | $-(CH_2)_4-O-(CH_2)_2CH(CH_3)(CH_2)_3CH(CH_3)(CH_2)_3CH(CH_3)_2$ | $-CH_2C_6H_5$ | Br | |
| 125 | $-(CH_2)_4-O-(CH_2)_2CH(CH_3)(CH_2)_3CH(CH_3)(CH_2)_3CH(CH_3)_2$ | $-CH_2C_6H_5$ | Cl | |
| 126 | $-(CH_2)_4-O-(CH_2)_2CH(CH_3)(CH_2)_3CH(CH_3)(CH_2)_3CH(CH_3)_2$ | $-CH_2CH=CH_2$ | Br | |
| 127 | $-(CH_2)_4-O-(CH_2)_2CH(CH_3)(CH_2)_3CH(CH_3)(CH_2)_3CH(CH_3)_2$ | $-CH_2CH_2O-C_6H_5$ | Br | |
| 128 | $-(CH_2)_4O-(2-CH(CH_3)_2-5-CH_3-cycl.C_6H_9)$ | $CH_2C_6H_5$ | Br | 135 |
| 129 | $-(CH_2)_4O-(2-CH(CH_3)_2-5-CH_3-cycl.C_6H_9)$ | $CH_3$ | Br | |
| 130 | $-(CH_2)_4O-(2-CH(CH_3)_2-5-CH_3-cycl.C_6H_9)$ | $CH_3$ | I | |
| 131 | $-(CH_2)_4O-(2-CH(CH_3)_2-5-CH_3-cycl.C_6H_9)$ | $-CH_2-CH=CH_2$ | Br | |
| 132 | $-(CH_2)_6-O-(4-tert.C_4H_9-cycl.C_6H_{10})$ | $-CH_3$ | Br | |
| 133 | $-(CH_2)_6-O-(4-tert.C_4H_9-cycl.C_6H_{10})$ | $-CH_3$ | I | 96 |
| 134 | $-(CH_2)_6-O-(4-tert.C_4H_9-cycl.C_6H_{10})$ | $-CH_2C_6H_5$ | Br | 116 |
| 135 | $-(CH_2)_6-O-(4-tert.C_4H_9-cycl.C_6H_{10})$ | $-CH_2-C_6H_5$ | Cl | |
| 136 | $-(CH_2)_6-O-(4-tert.C_4H_9-cycl.C_6H_{10})$ | $-CH_2-CH=CH_2$ | Br | |
| 137 | $-(CH_2)_4-O-CH_2CH(CH_3)CH_2-C_6H_5$ | $-CH_3$ | I | Öl |
| 138 | $-(CH_2)_4-O-CH_2CH(CH_3)CH_2-C_6H_5$ | $-CH_3$ | Br | |
| 139 | $-(CH_2)_4-O-CH_2CH(CH_3)CH_2-C_6H_5$ | $-CH_2C_6H_5$ | Br | 110 |
| 140 | $-(CH_2)_4-O-CH_2CH(CH_3)CH_2-C_6H_5$ | $-CH_2C_6H_5$ | Cl | |
| 141 | $-(CH_2)_4-O-CH_2CH(CH_3)CH_2-C_6H_5$ | $-CH_2CH=CH_2$ | Br | |

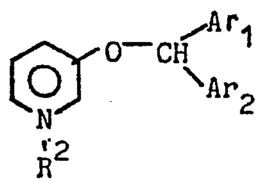

| Nr. | $Ar^1$ | $Ar^2$ | $R^2$ | X | Fp. [°C] |
|---|---|---|---|---|---|
| 142 | $-C_6H_5$ | $-C_6H_5$ | $-CH_3$ | I | |
| 143 | $-C_6H_5$ | $-C_6H_5$ | $-CH_3$ | Br | |
| 144 | $-C_6H_5$ | $-C_6H_5$ | $-CH_2.CH=CH_2$ | Br | |
| 145 | $-C_6H_5$ | $-C_6H_5$ | $-CH_2C_6H_5$ | Br | |
| 146 | $-C_6H_5$ | $-(4-Cl)C_6H_4$ | $-CH_3$ | I | 110 – 111 |
| 147 | $-C_6H_5$ | $-(4-Cl)C_6H_4$ | $-CH_3$ | Br | |
| 148 | $-C_6H_5$ | $-(4-Cl)C_6H_4$ | $-CH_2CH=CH_2$ | Br | |
| 149 | $-C_6H_5$ | $-(4-Cl)C_6H_4$ | $-CH_2C_6H_5$ | Br | Öl |
| 150 | $-(4-Cl)C_6H_4$ | $-(4-Cl)C_6H_4$ | $-CH_3$ | Br | |
| 151 | $-(4-Cl)C_6H_4$ | $-(4-Cl)C_6H_4$ | $-CH_3$ | I | 190 |
| 152 | $-(4-Cl)C_6H_4$ | $-(4-Cl)C_6H_4$ | $-CH_2CH=CH_2$ | Br | |
| 153 | $-(4-Cl)C_6H_4$ | $-(4-Cl)C_6H_4$ | $-CH_2C_6H_5$ | Br | 71 |
| 154 | $-C_6H_5$ | $-(3-CF3)C_6H_4$ | $-CH_3$ | Br | |
| 155 | $-C_6H_5$ | $-(3-CF3)C_6H_4$ | $-CH_3$ | I | 161 |
| 156 | $-C_6H_5$ | $-(3-CF3)C_6H_4$ | $-CH_2CH=CH_2$ | Br | |
| 157 | $-C_6H_5$ | $-(3-CF3)C_6H_4$ | $-CH_2C_6H_5$ | Br | Öl |
| 158 | $-(4-F)C_6H_4$ | $-(2,4-Cl2)C_6H_3$ | $-CH_3$ | Br | |
| 159 | $-(4-F)C_6H_4$ | $-(2,4-Cl2)C_6H_3$ | $-CH_3$ | I | 65 – 66 |
| 160 | $-(4-F)C_6H_4$ | $-(2,4-Cl2)C_6H_3$ | $-CH_2-CH=CH_2$ | Br | |
| 161 | $-(4-F)C_6H_4$ | $-(2,4-Cl2)C_6H_3$ | $-CH_2C_6H_5$ | Br | 90 |
| 162 | $-(2-F)C_6H_4$ | $-(4-F)C_6H_4$ | $-CH_3$ | Br | |
| 163 | $-(2-F)C_6H_4$ | $-(4-F)C_6H_4$ | $-CH_3$ | I | 143 |
| 164 | $-(2-F)C_6H_4$ | $-(4-F)C_6H_4$ | $-CH_2CH=CH_2$ | Br | |
| 165 | $-(2-F)C_6H_4$ | $-(4-F)C_6H_4$ | $-CH_2C_6H_5$ | Br | 173 |

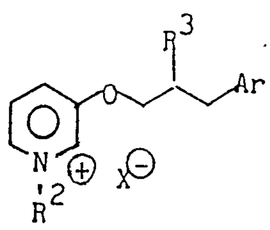

| Nr. | Ar | $R^2$ | $R^3$ | X | Fp. [°C] |
|---|---|---|---|---|---|
| 166 | $-(4-Cl)C_6H_4$ | $CH_3$ | $CH_3$ | Br | |
| 167 | $-(4-Cl)C_6H_4$ | $CH_3$ | $CH_3$ | I | |
| 168 | $-(4-Cl)C_6H_4$ | $-CH_2-CH=CH_2$ | $CH_3$ | Br | |
| 169 | $-(4-Cl)C_6H_4$ | $-CH_2C_6H_5$ | $CH_3$ | Br | |
| 170 | $-(4-C(CH_3)_3)C_6H_4$ | $CH_3$ | $CH_3$ | Br | |
| 171 | $-(4-C(CH_3)_3)C_6H_4$ | $CH_3$ | $CH_3$ | I | |
| 172 | $-(4-C(CH_3)_3)C_6H_4$ | $-CH_2CH=CH_2$ | $CH_3$ | Br | |
| 173 | $-(4-C(CH_3)_3)C_6H_4$ | $-CH_2C_6H_5$ | $CH_3$ | Br | |

| Verbindung Nr. | Lösungsmittel | Chem. Verbindung in ppm |
|---|---|---|
| 96 | $d_6$-DMSO | s 5,9 [24]; verbr. dd 8,15 [1H]; verbr. d 8,30 [14], d 8,90 [1H]; s 9,25 [1H] |
| 119 | $d_6$-DMSO | s 5,92 [2H]; dd 8,10 [1H]; verbr. d 8,28 [1H]; d 8,92 [1H]; s 9,28 [1H] |
| 137 | $d_6$-DMSO | s 4,35 [3H]; dd 8,05 [1H]; verbr. d 8,20 [1H]; d 8,62 [1H]; s 8,92 [1H] |
| 149 | $CDCl_3$ | s 6,15 [2H]; verbr. dd 7,79 [1H]; verbr. d 7,98 [1H]; d 9,09 [1H]; s 9,52 [1H] |
| 157 | $d_6$-DMSO | s 5,85 [2H]; dd 8,10 [1H]; verbr. d 8,35 [1H; d 8,90 [1H]; s 9,40 [1H] |

Die Wirkstoffe dienen zur Verhütung und Heilung von Pflanzenkrankheiten, die durch phytopathogene Pilze verursacht werden, insbesondere aus der Klasse der Ascomyceten und Phycomyceten aus. Sie sind zum Teil systemisch wirksam und können als Blatt- und Bodenfungizide eingesetzt werden.

Besonders interessant sind die fungiziden Verbindungen für die Bekämpfung von Pflanzenkrankheiten an einer Vielzahl von verschiedenen Kulturpflanzen oder ihren Samen, insbesondere Weizen, Roggen. Gerste, Hafer, Reis, Mais, Baumwolle, Soja, Kaffee, Zuckerrohr, Obst und Zierpflanzen im Gartenbau, Weinbau sowie Gemüse – wie Gurken, Bohnen und Kürbisgewächse.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrohr,
Venturia inaequalis (Schorf) an Äpfeln,
Septoria nodorum an Weizen,
Pyrenophora teres an Gerste,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Alternaria solani an Kartoffeln, Tomaten,
Plasmopara viticola an Reben sowie Fusarium- und Verticillium-Arten an verschieden Pflanzen

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung oder wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha. Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 11 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 12 werden in einer Mischung gelöst, die aus 80 Gewichtsteilen

Xylol, 10 Gewichtsteilen des Anlagerungsproduktes von 8 bis 10 Mol Ölsäure-N-monoethanolamid, 5 Gewichtsteilen Calciumsalz der Dodecylbenzolsulfonsäure und 5 Gewichtsteilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 13 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 14 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 23 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

VI. 3 Gewichtsteile der Verbindung Nr. 25 werden mit 97 Gewichtsteilen feinteiligem Kaolin innig vermischt. Man erhält auf diese Weise ein Stäubemittel, das 3 Gew.-% des Wirkstoffs enthält.

VII. 30 Gewichtsteile der Verbindung Nr. 28 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 29 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 30 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungs-gemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat
Tetramethylthiuramdisulfide,
Ammoniak-Komplex von Zink-(N,N-ethylen-bis-dithiocarbamat)
Ammoniak-Komplex von Zink-(N,N'-propylen-bis-dithiocarbamat),
Zink-(N,N'-propylen-bis-dithiocarbamat),
N,N'-Propylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester,

heterocyclische Strukturen, wie
2-Heptadecyl-2-imidazolin-acetat,
2,4-Dichlor-6-(o-chloranilino)-s-triazin,
0,0-Diethyl-phthalimidophosphonothioat,
5-Amino-1-(bis-(dimethylamino)-phosphinyl)-3-phenyl-1,2,4-triazol,
2,3-Dicyano-1,4-dithioanthrachinon,

2-Thio-1,3-dithio-(4,5-b)-chinoxalin,
1-(Butylcarbamoyl)-2-benzimidazol-carbaminsäuremethylester,
2-Methoxycarboxylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid;

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol
5-Butyl-2-dimethylamino-4-hydroxy-6-methyl-pyrimidin,
Bis-(p-Chlorphenyl)-pyridinmethanol,
1,2-Bis-(3-ethoxycarbonyl-2-thioureido)-benzol,
1,2-Bis-(3-methoxycarbonyl-2-thioureido)-benzol

sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,

DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-(3,5-Dichlorphenyl(5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureamid
2-Cyano-N-(ethylaminocarbonyl)-2-methoximino)-acetamid
1-(2-(2,4-Dichlorphenyl)-pentyl)-1H-1,2,4-triazol
2,4-Difluor-α-(1H-1,2,4-triazolyl-1-methyl)-benzhydrylalkohol
N-(n-Propyl)-N-(2,4,6-trichlorphenoxyethyl)-N'-imidazolylharnstoff

Für die folgenden Versuche wurde als Vergleich der bekannte Wirkstoff N-Trichlormethylthiotetrahydrophthalimid (A) verwendet.

Anwendungsbeispiel 1

Wirksamkeit gegen Botrytis cinerea an Paprika

Paprikasämlinge der Sorte "Neusiedler Ideal Elite" werden, nachdem sich 4–5 Blätter gut entwickelt haben, mit wäßrigen Suspensionen, die 80% Wirkstoff und 20% Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22–24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß die Verbindungen 11, 12, 13, 14, 23, 25, 27, 28, 29, 30, 40 und 41 bei der Anwendung als 0,05%ige (Gew.%) Spritzbrühe eine bessere fungizide Wirkung haben (z.B. 97%) als der Wirkstoff A (70%).

Anwendungsbeispiel 2

Wirksamkeit gegen Phytophthora infestans an Tomaten

Blätter von Topfpflanzen der Sorte "Große Fleischtomate" werden mit wäßriger Spritzbrühe, die 80% Wirkstoff und 20% Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß die Verbindungen 6, 9, 11, 12, 13, 14, 22, 23, 25, 27, 30, 32, 40, 41, 60 und 61 bei der Anwendung als 0,025%ige Spritzbrühe eine bessere fungizide Wirkung haben (90%) als der Wirkstoff A (60%).

Anwendungsbeispiel 3

Wirksamkeit gegen Pyricularia oryzae (protektiv)

Blätter von in Töpfen gewachsenen Reiskeimlingen der Sorte "Bahia" werden mit wäßrigen Emulsionen, die 80% Wirkstoff und 20% Emulgiermittel in der Trockensubstanz enthalten, tropfnaß besprüht und 24 Stunden später mit einer wäßrigen Sporensuspension von Pyricularia oryzae inoculiert. Anschließend werden die Versuchspflanzen in Klimakammern bei 22–24°C und 95–99% relativer Luftfeuchtigkeit aufgestellt. Nach 6 Tagen wird das Ausmaß des Krankheitsbefalls ermittelt.

Das Ergebnis des Versuches zeigt, daß die Verbindungen 5, 7, 23 und 40 bei der Anwendung als 0,05%ige Spritzbrühe ein gute fungizide Wirkung haben (z.B. 97%).

Anwendungsbeispiel 4

Wirksamkeit gegen Septoria nodorum

Blätter von in Töpfen gewachsenen Weizenkeimlingen der Sorte "Jubilar" werden mit wäßriger Spritzbrühe, die 80% Wirkstoff und 20% Emulgiermittel in der Trockensubstanz enthält, bis zur Tropfnässe besprüht. Am folgenden Tag werden die angetrockneten Pflanzen mit einer wäßrigen Sporensuspension von Septoria nodorum infiziert und dann für 10 Tage bei 17–19°C und 95%iger relativer Luftfeuchtigkeit weiter kultiviert. Das Ausmaß des Pilzbefalls wrid dann visuell ermittelt.

Das Ergebnis des Versuchs zeigt, daß die Verbindungen 5, 7, 23, 40, 60, 61, 146, 149, 157, 159, 161 und 163 eine gute fungizide Wirkung haben (z.B. 97%).

**Patentansprüche**

1. O-substituierte 3-Oxypyridiniumsalze der Formel

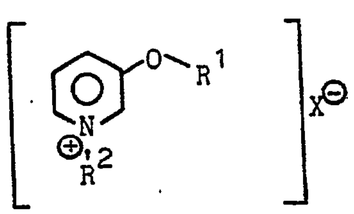

I,

in der

$R^1$ einen gegebenenfalls durch Alkoxy oder Halogen substituierten Alkyl-, Alkenyl- oder Alkinylrest mit insgesamt 8– 20 C-Atomen oder eine der Gruppen

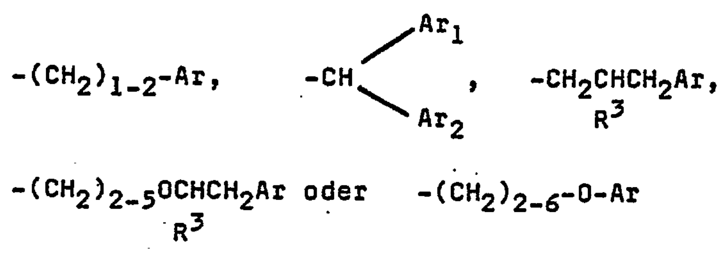
oder

bedeutet, wobei Ar, $Ar_1$ und $Ar_2$ für 1- oder 2-Naphthyl, Biphenyl oder Phenyl steht und der Phenylrest durch F, Cl, Br, $NO_2$, $CF_3$, CN, $C_{1-13}$-Alkyl, $C_{2-4}$-Alkenyl oder $C_{1-2}$-Alkoxy substituiert sein kann,

$R^3$ H, $C_{1-2}$-Alkyl, $C_{3-5}$-Alkenyl, oder $C_1$–$C_5$-Alkoxy bedeutet und

$R^2$ einen gegebenenfalls durch $C_1$–$C_4$-Alkoxy oder Halogen substituierten Alkyl-, Alkenyl- oder Alkinylrest mit 1 bzw. 2 oder 3 bis 8 Kohlenstoffatomen oder eine der Gruppen

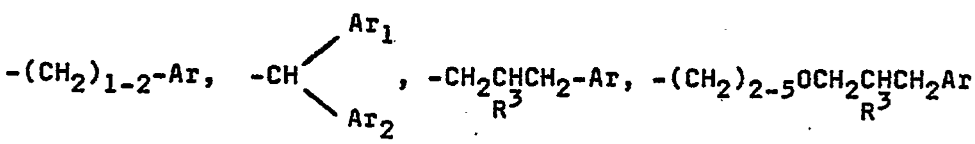

oder $-(CH_2)_{2-6}$–O–Ar bedeutet, wobei Ar, $Ar_1$ und $Ar_2$ für 1- oder 2-Naphthyl, Biphenyl oder Phenyl steht und der Phenylrest durch F, Cl, Br, $NO_2$, $CF_3$, CN, $C_1$–$C_{13}$-Alkyl, $C_2$–$C_4$-Alkenyl oder $C_1$–$C_2$-Alkoxy substituiert sein kann und

$X^\ominus$ $F^\ominus$, $Cl^\ominus$, $Br^\ominus$, $J^\ominus$ oder ein Anion, das ein Äquivalent einer nicht phytotoxischen Säure ist, bedeutet.

2. O-substituierte 3-Oxypyridiniumsalze der Formel I gemäß Anspruch 1, dadurch gekennzeichnet, daß $R^1$ gerades oder verzweigtes, gegebenenfalls durch 2–5 Cl- oder F-Atome substituiertes Decyl, Undecyl, Tridecyl, Tetradecyl, Pentadecyl, Hexadecyl, Heptadecyl, Octadecyl oder die Gruppe $C_{2-10}$-Alkyl-O-$C_{5-18}$-Alkyl mit insgesamt 10 bis 20 C-Atomen und gegebenenfalls mit 1 bis 2 Doppelbindungen oder einer Dreifachbindung oder die Reste

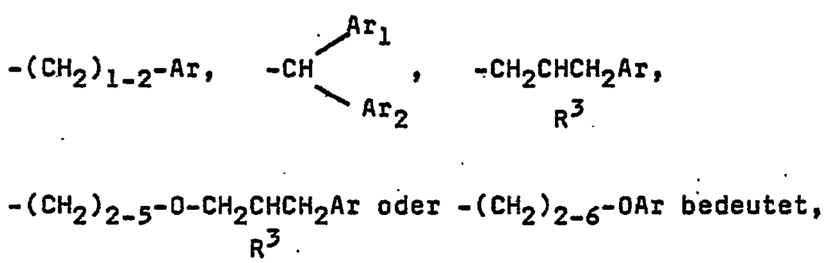
oder
bedeutet,

wobei Ar, $Ar_1$ und $Ar_2$ für 1- oder 2-Naphthyl, Biphenyl, Phenyl, einfach bis dreifach substituiertes Phenyl, 4-Fluorphenyl, 3- oder 4-Chlorphenyl, 4-Bromphenyl, 3,4-Dichlorphenyl, 2,4-Dichlorphenyl, 2,6-Dichlorphenyl, 2,4,6-Trichlorphenyl, 2-Methyl-4-Chlorphenyl, 4-Methoxyphenyl, 4-Ethoxyphenyl, 4-Methylphenyl, 4-Nitrophenyl, 4-tert.-Butylphenyl, 4-Dodecylphenyl, 4-Trifluormethylphenyl, 2-Trifluormethyl-4-chlorphenyl, 2,4-Dimethylphenyl, 2,4,6-Trimethylphenyl und 4-Cyanophenyl steht,

$R^3$ Wasserstoff, Methyl oder Ethyl bedeutet,

$R^2$ Methyl, Ethyl, Propyl, Isopropyl, n-Butyl, Isobutyl, n-Pentyl, Isopentyl, n-Hexyl, Allyl, 2-Chlorpropenyl, 2-Brompropenyl, 3-Chlorpropenyl, 2-Buten-1-yl, 3-Methyl-2-buten-1-yl, 2-Methylpropenyl, 3-Chlor-2-buten-1-yl, Propargyl oder die Reste

$-(CH_2)_{1-2}-Ar$, 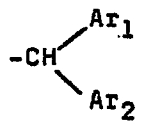 , $-CH_2\underset{R^3}{CH}CH_2Ar$,

$-(CH_2)_{2-5}-O-CH_2\underset{R^3}{CH}CH_2Ar$ oder $-(CH_2)_{2-6}-OAr$ bedeutet,

oder $-(CH_2)_{2-6}OAr$ bedeutet,

wobei Ar, $Ar_1$ und $Ar_2$ für 1- und 2-Naphthyl, Biphenyl, Phenyl, einfach bis dreifach substituiertes Phenyl, 4-Fluorphenyl, 3- oder 4-Chlorphenyl, 4-Bromphenyl, 3,4-Dichlorphenyl, 2,4-Dichlorphenyl, 2,6-Dichlorphenyl, 2,4,6-Trichlorphenyl, 2-Methyl-4-Chlorphenyl, 4-Methoxyphenyl, 4-Ethoxyphenyl, 4-Methylphenyl, 4-Nitrophenyl, 4-tert.-Butylphenyl, 4-Dodecylphenyl, 4-Trifluormethylphenyl, 2-Trifluormethyl-4-Chlorphenyl, 2,4-Dimethylphenyl, 2,4,6-Trimethylphenyl und 4-Cyanophenyl steht, und
$X^-$ $Cl^-$, $Br^-$ oder $I^-$ oder ein Anion, das ein Äquivalent einer nicht phytoxischen Säure ist, bedeutet.

3. Verfahren zur Herstellung von O-substituierten 3-Oxypyridiniumsalzen gemäß Anspruch 1, dadurch gekennzeichnet, daß man Verbindungen der Formel
$R^2X$ II,
in der $R^2$ und X die im Anspruch 1 angegebenen Bedeutungen haben, mit einem Pyridinderivat der Formel

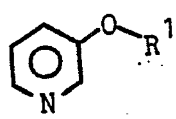 III,

in der $R^1$ die im Anspruch 1 angegebene Bedeutung hat, umsetzt.

4. Fungizides Mittel für den Pflanzenschutz, enthaltend einen festen oder flüssigen Trägerstoff und ein O-substituiertes 3-Oxypyridiniumsalz gemäß Anspruch 1.

5. Verfahren zur Bekämpfung von phytopathogenen Pilzen, dadurch gekennzeichnet, daß man ein O-substituiertes 3-Oxypyridiniumsalz gemäß Anspruch 1 auf Pilze oder auf durch Pilzbefall bedrohte Pflanzen oder Saatgüter einwirken läßt.

6. Verfahren zur Herstellung eines fungiziden Mittels für den Pflanzenschutz, dadurch gekennzeichnet, daß man ein O-substituiertes 3-Oxypyridiniumsalz gemäß Anspruch 1 mit festen oder flüssigen Trägerstoffen sowie gegebenenfalls mit einem oder mehreren oberflächenaktiven Mitteln mischt.

**Revendications**

1. Sels de 3-oxypyridinium substitué sur O, de formule

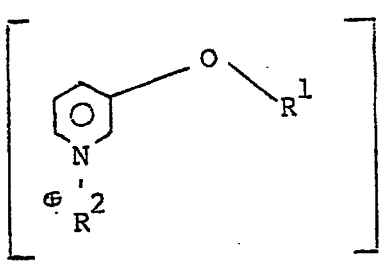 $X^{\ominus}$ I,

dans laquelle
$R_1$ représente un reste alkyle, alcényle ou alcinyle ayant au total 8–20 atomes C, éventuellement substitué par alcoxy ou halogène, ou un des groupes

$-(CH_2)_{1-2}-Ar$, 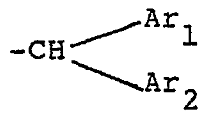 , $-CH_2\underset{R^3}{CH}CH_2Ar$,

$-(CH_2)_{2-5}O\underset{R^3}{CH}CH_2Ar$ ou $-(CH_2)_{2-6}-O-Ar$

Ar, $Ar_1$ et $Ar_2$ étant mis pour 1- ou 2-naphtyle, biphényle ou phényle et le reste phényle pouvant être substitué par F, Cl, Br, $NO_2$, $CF_3$, CN, alkyle en $C_{1-13}$, alcényle en $C_{2-4}$ ou alcoxy en $C_{1-2}$
$R^3$ représente H, alkyle en $C_{1-2}$, alcényle en $C_{3-5}$ ou alcoxy en $C_{1-2}$ et
$R^2$ représente un reste alkyle, alcényle, ou alkinyle de 1 et 2 ou 3 à 8 atomes de carbone, eventuellement substitué par alcoxy en $C_{1-4}$ ou halogène, ou un des groupes

$-(CH_2)_{1-2}-Ar$, 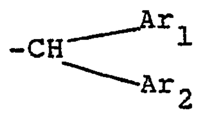 ; $-CH_2\underset{R^3}{CH}CH_2-Ar$ , $-(CH_2)_{2-5}OCH_2\underset{R^3}{CH}CH_2Ar$
ou $-(CH_2)_{2-6}-O-Ar$

Ar, $Ar_1$ et $Ar_2$ étant mis pour 1- ou 2-naphtyle, biphényle ou phényle et le reste phényle pouvant être substitué par F, Cl, Br, $NO_2$, $CF_2$, CN, alkyle en $C_{1-13}$, alcényle en $C_{2-4}$ ou alcoxy en $C_{1-2}$ $X^{\ominus}$ représente $F^{\ominus}$, $Cl^{\ominus}$, $Br^{\ominus}$, $I^{\ominus}$ ou un anion qui est un équivalent d'un acide non phytotoxique.

2. Sels de 3-oxypyridinium substitué sur O, de formule 1 selon la revendication 1, caracterisé par le fait que
R1 représente décyle, undécyle, tridécyle, tetradécyle, pentadecyle, hexadecyle, hexadécyle, heptadécyle, octadécyle, linéaire ou ramifié, éventuellement substitué par des atomes 2–5 $C_l$ ou F, ou le groupe alkyle en $C_{2-10}$-O-alkyle en $C_{5-18}$ ayant au total 10 à 20 atomes C et éventuellement 1 à 2 doubles liaisons ou une triple liaison, ou les restes

$-(CH_2)_{1-2}-Ar$, 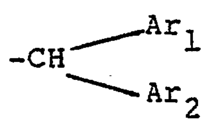 , $-CH_2\underset{R^3}{CH}CH_2Ar$,

$-(CH_2)_{2-5}-O-CH_2\underset{R^3}{CH}CH_2Ar$ ou $-(CH_2)_{2-6}-OAr$

Ar, $Ar_1$ et $Ar_2$ étant mis pour 1 ou 2-naphtyle, biphényle, phényle, phényle substitué une fois à trois fois, 4-fluorophényle, 3- ou 4-chlorophényle, 4-bromophényle, 3-4-dichlorophényle, 2,4-dichlorophényle, 2,6-dichlorophényle, 2,4,6-trichlorophényle, 2-méthyl-4-chlorophényle, 4-methoxyphényle, 4-ethoxyphényle, 4-méthylphényle, 4-nitrophényle, 4-tert.-butylphényle, 4-dodecylphényle, 4-trifluorméthylphényle, 2-trifluorméthyl-4-chlorophényle, 2,4-diméthylphényle, 2,4,6-trimethylphényle et 4-cyanophenyle
$R_3$ représente hydrogène, méthyle ou éthyle,
$R_2$ représente méthyle, éthyle, propyle, isopropyle, n-butyle, isobutyle, n-pentyle, isopentyle, n-héxyle, allyle, 2-chloropropenyle, 2-bromopropenyle, 3-chloropenyle, 2-buten-1-yle, 3-méthyl-2-buten-1-yle, 2-méthylpropenyle, 3-chloro-2-buten-1-yle, propargyle ou les restes

$-(CH_2)_{1-2}-Ar$, 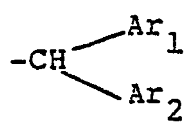 , $-CH_2\underset{R^3}{CH}CH_2Ar$,

$$-(CH_2)_{2-5}-O-CH_2\underset{R^3}{CH}CH_2Ar$$

$$\text{ou}\quad -(CH_2)_{2-6}OAr$$

Ar, $Ar_1$ et $Ar_2$ étant mis pour 1 ou 2-naphtyle, biphényle, phényle, phényle substitué une fois à trois fois, 4-fluorophényle, 3- ou 4-chlorophényle, 4-bromophényle, 3-4-dichlorophényle, 2,4-dichlorophényle, 2,6-dichlorophényle, 2,4,6-trichlorophényle, 2-méthyl-4-chlorophényle, 4-methoxyphényle, 4-ethoxyphényle, 4-méthylphényle, 4-nitrophényle, 4-tert.-butylphényle, 4-dodecylphényle, 4-trifluorométhylphényle, 2-trifluorométhyl-4-chlorophényle, 2,4-diméthylphényle, 2,4,6-trimethylphényle et 4-cyanophenyle, et

$X^\ominus$ représente $Cl^\ominus$, $Br^\ominus$ ou $I^\ominus$ ou un anion qui est un équivalent d'un acide non phytotoxique.

3. Procédé de préparation de sels de 3-oxypyridinium substitué sur O selon la revendication 1 caractérisé par le fait qu'on fait réagir des composés de formule

$R^2X$ II,

dans laquelle $R^2$ et X ont les significations données dans la revendications 1, avec un dérivé de pyridine de formule

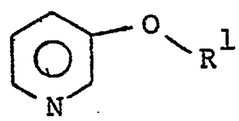

III,

dans laquelle $R_1$ a la signification donnée dans la revendication 1.

4. Fongicide pour la protection des plantes contenant un support solide ou liquide et un sel de 3-oxypyridinium substitué sur O selon la revendication 1.

5. Procédé de lutte contre les champignons phytopathogènes, caractérisé par le fait qu'on fait agir un sel de 3-oxypyridinium substitué sur O selon la revendication 1 sur les champignons ou sur les plantes ou semences menacés par les champignons.

6. Procédé de préparation d'un fongicide pour la protection des plantes, caractérisé par le fait que l'on mélange un sel de 3-oxypyridinium substitué sur O selon la revendication 1 avec un support solide ou liquide ainsi qu'éventuellement avec un ou plusieurs agents tensioactifs.

## Claims

1. An O-substituted 3-oxypyridinium salt of the formula

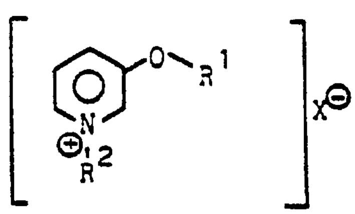

I

where $R^1$ is an alkyl, alkenyl or alkynyl radical which contains a total of 8 to 20 carbon atoms and is unsubstituted or substituted by alkoxy or halogen, or $R^1$ is one ot fhe groups

$-(CH_2)_{1-2}-Ar$, 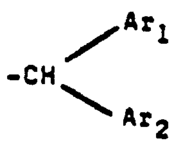, $-CH_2\underset{R^3}{CH}CH_2Ar$,

$-(CH_2)_{2-5}O\underset{R^3}{CH}CH_2Ar$ or $-(CH_2)_{2-6}-O-Ar$

where Ar, $Ar_1$ and $Ar_2$ are each 1- or 2-naphthyl, biphenyl or phenyl, and the phenyl radical may be substituted by F, Cl, Br, $NO_2$, $CF_3$, CN, $C_1$–$C_{13}$-alkyl, $C_2$–$C_4$-alkenyl or $C_1$- or $C_2$-alkoxy, $R^3$ is H, $C_1$–$C_2$-alkyl, $C_3$–$C_5$-alkenyl or $C_1$–$C_5$-alkoxy and $R^2$ is an alkyl, alkenyl or alkynyl radical of 1 or 2 or 3 to 8 carbon atoms which is unsubstituted or substituted by $C_1$–$C_4$-alkoxy or halogen, or $R^2$ is one of the groups

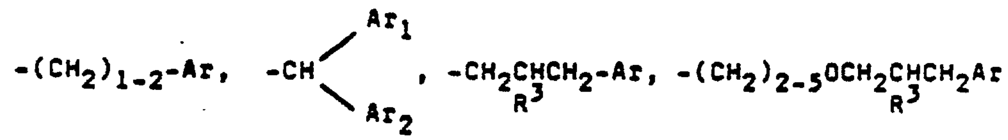

or $-(CH_2)_{2-6}$-O-Ar, where Ar, $Ar_1$ and $Ar_2$ are each 1- or 2-naphthyl, biphenyl or phenyl, and the phenyl radical may be substituted by F, Cl, Br, $NO_2$, $CF_3$, CN, $C_1$–$C_{13}$-alkyl, $C_2$–$C_4$-alkenyl or $C_1$–$C_2$-alkoxy, and $X^\ominus$ is $F^\ominus$, $Cl^\ominus$, $Br^\ominus$, $I^\ominus$ or one equivalent of an anion of a nonphytotoxic acid.

2. An O-substituted 3-oxypyridinium salt of the formula I as claimed in claim 1, wherein $R^1$ is undecyl, tridecyl, tetradecyl, pentadecyl, hexadecyl, heptadecyl, octadecyl or decyl which is straight-chain or branched and unsubstituted or substituted by 2–5 Cl or F atoms, or is a group $C_{2-10}$-alkyl-O-$C_{5-18}$-alkyl which contains a total of 10 to 20 carbon atoms and may contain 1 or 2 double bonds or one triple bond, or is the radical

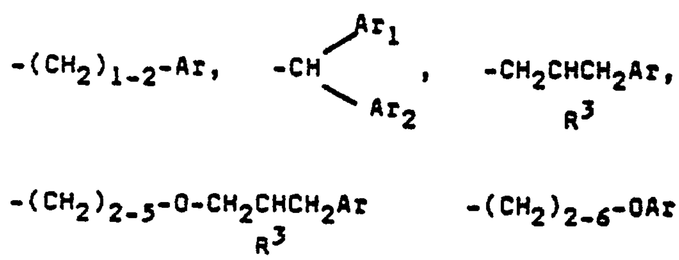

where Ar, $Ar_1$ and $Ar_2$ are each 1- or 2-naphthyl, biphenyl, phenyl, monosubstituted, disubstituted or trisubstituted phenyl, 4-fluorophenyl, 3- or 4-chlorophenyl, 4-bromophenyl, 3,4-dichlorophenyl, 2,4-dichlorphenyl, 2,6-dichlorophenyl, 2,4,6-trichlorophenyl, 2-methyl-4-chlorophenyl, 4-methoxyphenyl, 4-ethoxyphenyl, 4-methylphenyl, 4-nitrophenyl, 4-tert-butylphenyl, 4-dodecylphenyl, 4-trifluoromethylphenyl, 2-trifluoromethyl-4-chlorophenyl, 2,4-dimethylphenyl, 2,4,6-trimethylphenyl or 4-cyanophenyl, $R^3$ is hydrogen, methyl or ethyl, $R^2$ is methyl, ethyl, propyl, isopropyl, n-butyl, isobutyl, n-pentyl, isopentyl, n-hexyl, allyl, 2-chloropropenyl, 2-bromopropenyl, 3-chloropropenyl, 2-buten-1-yl, 3-methyl-2-buten-1-yl, 2-methylpropenyl, 3-chloro-2-buten-1yl, propargyl or a radical

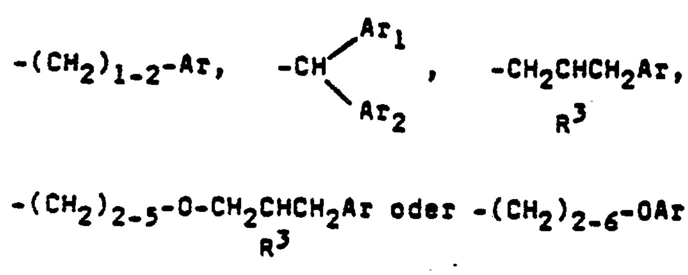

where Ar, $Ar_1$ and $Ar_2$ are each 1- or 2-naphthyl, biphenyl, phenyl, monosubstituted, disubstituted or trisubstituted phenyl, 4-fluorophenyl, 3- or 4-chlorophenyl, 4-bromophenyl, 3,4-dichlorophenyl, 2,4-dichlorophenyl, 2,6-dichlorophenyl, 2,4,6-trichlorophenyl, 2-methyl-4-chlorophenyl, 4-methoxyphenyl, 4-ethoxyphenyl, 4-methylphenyl, 4-nitrophenyl, 4-tert-butylphenyl, 4-dodecylphenxl, 4-trifluoromethylphenyl, 2-trifluoromethyl-4-chlorophenyl, 2,4-dimethylphenyl, 2,4,6-trimethylphenyl or 4-cyanophenyl, and

$X^\ominus$ is $F^\ominus$, $Cl^\ominus$, $Br^\ominus$ or$I^\ominus$ or one equivalent of an anion of a nonphytotoxic acid.

3. A process for the preparation of an O-substituted 3-oxypyridinium salt as claimed in claim 1, wherein a compound of the formula

$R^2X$ II,

where $R^2$ and X have the meanings stated in claim 1, is reacted with a pyridine derivative of the formula

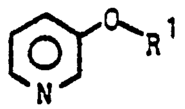

III

where $R^1$ has the meanings stated in claim 1.

4. A fungicide for crop protection, containing a solid or liquid carrier and an O-substituted 3-oxypyridinium salt as claimed in claim 1.

5. A method for controlling phytopathogenic fungi, wherein an O-substituted 3-oxypyridinium salt as claimed in claim 1 is allowed to act on fungi or on plants or seed threatened by fungal attack.

6. A process for the preparation of a fungicide for crop protection, wherein an O-substituted 3-oxypyridinium salt as claimed in claim 1 is mixed with solid or liquid carriers and, if necessary, with one or more surfactants.